(19) Europäisches Patentamt
European Patent Office
Office européen des brevets

(11) Publication number: **0 089 697**
**B1**

# (12) EUROPEAN PATENT SPECIFICATION

(45) Date of publication of patent specification: **17.09.86**

(51) Int. Cl.$^4$: **C 07 F 15/00,** B 01 J 31/18

(21) Application number: **83103487.1**

(22) Date of filing: **03.10.79**

(60) Publication number of the earlier application in accordance with Art. 76 EPC: **0 014 796**

## (54) Rhodium compounds and process for producing them.

(30) Priority: **04.10.78 JP 121456/78**
**04.10.78 JP 121457/78**
**06.04.79 JP 40994/79**

(43) Date of publication of application:
**28.09.83 Bulletin 83/39**

(45) Publication of the grant of the patent:
**17.09.86 Bulletin 86/38**

(84) Designated Contracting States:
**BE DE FR GB IT NL**

(56) References cited:
**GB-A-1 357 735**
**US-A-3 527 809**
**US-A-3 821 311**
**US-A-3 857 900**

(73) Proprietor: **TOA NENRYO KOGYO KABUSHIKI KAISHA**
**1-1 Hitotsubashi, 1-Chome Chiyoda-Ku**
**Tokyo 100 (JP)**

(72) Inventor: **Sakakibara, Tadamori**
**1902-5 Ooaza Kamekubo**
**Ooi-machi Iruma-gun Saitama-ken (JP)**
Inventor: **Matsushima, Yoshihisa**
**1902-5 Ooaza Kamekubo**
**Ooi-machi Iruma-gun Saitama-ken (JP)**
Inventor: **Nagashima, Yukio**
**1906-199 Ooaza Kamekubo**
**Ooi-machi Iruma-gun Saitama-ken (JP)**
Inventor: **Okamoto, Nobukazu**
**1906-199 Ooaza Kamekubo**
**Ooi-machi Iruma-gun Saitama-ken (JP)**
Inventor: **Kaneko, Katsumi**
**151-108, Kume**
**Tokorozawa-shi Saitama-ken (JP)**
Inventor: **Ishii, Yoshio**
**93 Tokugawayama-cho 3-chome**
**Chikusa-ku Nagoya-shi Aichi-ken (JP)**
Inventor: **Wada, Shozo**
**7-5 Yamanone 2-chome**
**Zushi-shi Kanagawa-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: **Dew, Melvyn John et al**
**Esso Chemical Ltd. Esso Chemical Research Centre P.O. Box 1**
**Abingdon Oxfordshire, OX13 6BB (GB)**

**Description**

This invention relates to new rhodium compounds and a process for producing the same. More particularly, it is concerned with new rhodium compounds having a structure isoelectronic to $HRh(CO)(PPh_3)_3$ and a process for the selective production of the same. Further, it relates to use of such products as catalysts in the oxo process.

Carbonyl tris (triphenylphosphine) rhodium hydride is known as a catalyst having activity in various chemical reactions for organic syntheses, for example, in hydroformylation. (Japanese Patent Publication No. 10730/1970 equivalent to U.S. Patent 3,527,809). See also U.S. Patent 3,821,311 and Canadian Patent 992,101. As rhodium compounds having a structure isoelectronic thereto are known carbonyl tris(triphenylphosphite) rhodium hydride $HRh(CO)(P(OPh)_3)_3$ (British Patent No. 1338225), carbonyl tris(triphenylarsine) rhodium hydride $HRh(CO)(AsPh_3)_3$ and carbonyl tris (triphenylarsite) rhodium hydride $HRh(CO)(As(OPh)_3)_3$ (British Patent No. 1357735). As to producing these compounds, some processes are known. In the case of $HRh(CO)(PPh_3)_3$, for example, there have been proposed (1) a process comprising reacting rhodium trichloride trihydrate, excess triphenylphosphine, an aqueous solution of formaldehyde and sodium borohydride in boiling ethanol (Journal of the Chemical Society (A), 1968, page 2660), (2) a process comprising reacting carbonyl bis(triphenylphosphine) rhodium chloride, excess triphenylphosphine and sodium borohydride in boiling ethanol (ibid) and (3) a process comprising contacting carbonyl bis(triphenylphosphine) rhodium chloride and excess triphenylphosphine with synthesis gas (mixture of $H_2$ and CO) at a temperature of at least 55°C under a pressure of at least 10 atm (~10 bar) (Japanese Patent Publication No. 17572/1978).

Referring in more detail to the abovementioned GB1357735, this is concerned with hydroformylation reactions in which the olefin to be hydroformylated is present in gaseous form, and in which (absent a solvent) a liquid or molten arsenic- or antimony-containing stabilising donor ligand is used as reaction medium; the catalyst is a hydrido carbonyl complex of rhodium including an arsenic- or antimony-containing stabilising donor ligand. Specifically mentioned as catalytic rhodium complexes are $RhH(CO)(AsPh_3)_3$, and it is preferred to employ as the reaction medium a stabilising donor ligand which is the same as that employed in the complex, e.g. molten $AsPh_3$. The complex may be produced in situ from compounds such as $RhX(CO)(AsR_3)_3$, $RhX(CO)(SbR_3)_3$, $RhX_3(AsR_3)_3$ or $RhX_3(SbR_3)_3$ wherein X may be halogen and R may be aryl, preferably in the presence of an organic base which can act as a hydrogen halide acceptor. Alternatively the complexes may be generated from rhodium compounds in other oxidation states e.g. $RhCl_3$, rhodium carbonyl, rhodium(II) carboxylate, rhodium(II) carbonyl carboxylate, rhodium oxides, or rhodium β-diketonates.

Abovementioned US 3 527 809 also relates to hydroformylation processes and discloses performing the reaction in the presence of a rhodium containing complex catalyst and a tertiary organo-containing free ligand. The complex may be rhodium complexed with carbon monoxide and a ligand containing a trivalent atom including e.g. phosphorus, arsenic and antimony; whilst the free ligand may be a tertiary organic phosphorus, arsenic or antimony compound. Specifically exemplified complexes are phosphorus containing complexes $HRh(CO(PPh_3)_3$ in combination with phosphorus containing free ligands $P(OPh)_3$ (Table VII). The patent also teaches the use of triphenylarsenic and triphenyl stibine as as the free ligand in associated rhodium complex catalyst systems. The catalyst complex may be produced by reacting a rhodium organic acid salt with the ligand, followed by reduction, or alternatively by heating a CO complex of rhodium with the ligand.

However, carbonyl tris(triphenylstibine) rhodium hydride $HRh(CO)(SbPh_3)_3$ having a structure isoelectronic to the above described rhodium compounds has not been synthesized and isolated. Moreover, as is evident from the foregoing description, all the rhodium compounds having the above described known structures contain respectively a single kind of ligand and those containing different kinds of mixed ligands, for example, phosphines and arsines in the same molecule, are not known, although some monovalent rhodium compounds containing mixed ligands in the same molecule are known, for example, carbonyl triphenylphosphine triphenylarsine rhodium chloride $RhCl(CO)(PPh_3)$ $(AsPh_3)$ (Journal of Organometallic Chemistry, Vol. 43, No. 2, 1972, page 425), carbonyl triphenylphosphine triphenylstibine rhodium chloride $RhCl(CO)(PPh_3)(SbPh_3)$ (ibid), carbonyl triphenylarsine triphenylstibine rhodium chloride $RhCl(CO)(AsPh_3)(SbPh_3)$ (ibid) and carbonyl (diethylphenylphosphine) (tri-o-toluylstibine) rhodium chloride $RhCl(CO)(PPhEt_2)(Sb(o{-}CH_2C_6H_4)_3)$ (Inorganic Chemistry, Vol. 15, No. 13, 1976, page 646). See also J. Chem. Soc. D1970, (17), 1077—8 (England); Inorg. Nucl. Chem. Lett. 1971, 7(9)877—9 (England); J. Chem. Soc. Dalton Trans. 1972 (19) 2161—9 (England) and U.S. Patent 3,459,780. As a method for synthesizing these compounds, it has been proposed to add to a binuclear rhodium compound $(RhCl(CO)L)_2$ wherein L is triphenylphosphine, triphenylarsine or triphenylstibine in warm benzene an ether solution of 2 mols (equimolar amount) of L' being triphenylphosphine, triphenylarsine or triphenylstibine but not the same as L (Journal of Organometallic Chemistry, Vol. 43, No. 2, 1972, page 425).

Other references of lesser interest, noted because they concern rhodium-containing compounds, are U.S. Patents 3,939,188 and 3,946,082, which relate to compounds containing reduced, zero valent rhodium and 4,052,461 which relates to ionic rhodium compounds but not the hydrides which are non-ionic.

However, mixed ligands-containing rhodium compounds having a structure isoelectronic to $HRh(CO)(PPh_3)_3$ or three stibines-coordinated rhodium compounds cannot be obtained, or mixtures of

various rhodium compounds are only obtained, by the above described method or other known methods and it is very difficult to isolate such compounds in a pure form.

Rhodium-phosphine type or rhodium-phosphite type complexes are known as catalysts for the hydroformylation of olefins. The stability of a rhodium catalyst is increased by modification with phosphine, arsine, or stibine, which permits practicing the oxo reaction at a rather low pressure.

According to Japanese Patent No. 903,326, straight chain-rich aldehydes are prepared at a low total pressure with a low partial pressure of carbon monoxide and a high partial pressure of hydrogen in the presence of a rhodium triarylphosphine catalyst and a triarylphosphine ligand in a large excess to the rhodium. However, this method has the disadvantage that the hydroformylation reaction rate of an olefin is markedly decreased because of using a ligand in a large excess to rhodium, and a considerable quantity of a paraffin is formed by the hydrogenation of the olefin ("Hydrocarbon Processing" (4) 112 (1970)) due to the reaction at a low total pressure with a low partial pressure of carbon monoxide and a high partial pressure of hydrogen.

Rhodium catalysts in combination with arsines or stibines instead of phosphines have been proposed, but the studies thereof have not been made so intensively because of their lower activity compared with tertiary phosphine-rhodium catalysts.

Applicants have made various efforts to obtain new rhodium compounds having a structure isoelectronic to $HRh(CO)(PPh_3)_3$ and, as a result, have succeeded in obtaining new rhodium compounds represented by the following general formula in high yield and in a pure form. Accordingly the present invention provides novel rhodium compounds represented by the general formula,

$$HRh(CO)(XR_3)_2(YR_3) \qquad (A)$$

wherein X and Y are different and are each phosphorus, arsenic or antimony; and R is phenyl. It is estimated that in the new compounds the rhodium is mono-valent.

Figures 1 and 2 are graphs showing the relationships between the sum of the Pauling electronegativities and $\upsilon_{C=O}$ and $\upsilon_{Rh-H}$ respectively of compounds of this invention.

The novel rhodium compounds included in the above described general formula (A) are as follows:

(1) Carbonyl bis(triphenylphosphine) triphenylarsine rhodium hydride $HRh(CO)(PPh_3)_2(AsPh_3)$

(2) Carbonyl bis(triphenylphosphine) triphenylstibine rhodium hydride $HRh(CO)(SbPh_3)(PPh_3)_2$

(3) Carbonyl bis(triphenylarsine) triphenylphosphine rhodium hydride $HRh(CO)(PPh_3)(AsPh_3)_2$

(4) Carbonyl bis(triphenylarsine) triphenylstibine rhodium hydride $HRh(CO)(SbPh_3)(AsPh_3)_2$

(5) Carbonyl bis(triphenylstibine) triphenylphosphine rhodium hydride $HRh(CO)(PPh_3)(SbPh_3)_2$

(6) Carbonyl bis(triphenylstibine) triphenylarsine rhodium hydride $HRh(CO)(AsPh_3)(SbPh_3)_2$.

Also mentioned here is

(7) Carbonyl tris(triphenylstibine) rhodium hydride $HRh(CO)(SbPh_3)_3$ which, as with (1)—(6), may be prepared by the process hereinafter described.

The structural formulas of these seven kinds of compounds were confirmed by an IR spectrophotometer and elementary analysis, results shown in Tables 1 and 2 being obtained. As can be seen from Table 1, the IR data of the compounds of the present invention show that the absorption based on the stretching frequency of Rh-H ($\upsilon_{Rh-H}$) appears near 2000 $cm^{-1}$, the absorption based on the stretching frequency of C=O ($\upsilon_{C=O}$) appears near 1900 $cm^{-1}$ and the absorption zones are substantially similar to those of $\upsilon_{Rh-H}$ and $\upsilon_{C=O}$ of the known compounds $HRh(CO)(PPH_3)_3$ and $HRh(CO)(AsPh_3)_3$. In addition, there is a linear relationship between the sum of the Pauling electro-negativities of the elements and $\upsilon_{C=O}$ or $\upsilon_{Rh-H}$ in these compounds, respectively as shown in Figure 1 and Figure 2. (In Figure 1 and Figure 2, $P_3$=$HRh(CO)(PPh_3)_3$*, $P_2As$=$HRh(CO)(PPh_3)_2(AsPh_3)$, $As_2P$=$HRh(CO)(AsPh_3)_2(PPh_3)$, $P_2Sb$=$HRh(CO)(PPh_3)_2(SbPh_3)$, $As_3$=$HRh(CO)(AsPh_3)_3$*, $Sb_2P$=$HRh(CO)(SbPh_3)_2(PPh_3)$, $As_2Sb$=$HRh(CO)(AsPh_3)_2(SbPh_3)$, $Sb_2As$=$HRh(CO)(SbPh_3)_2(AsPh_3)$, $Sb_3$=$HRh(CO)(SbPh_3)_3$, mark *=known compound). As apparent from Table 2, the found values in the elementary analysis of these compounds coincide exactly with calculated ones and thus it is confirmed that the compounds of the present invention have the designated general formula.

For the production of these compounds, the various known methods or the method comprising reacting a monovalent rhodium compound containing mixed ligands and excess ligands in the presence of sodium borohydride or hydrazine in boiling ethanol has the disadvantage set forth above that starting materials only are recovered, starting materials themselves are decomposed, or a mixture of various rhodium compounds is only obtained and isolation of the desired compound is very difficult.

According to a further aspect of the invention there is provided a process for producing a rhodium compound having the general formula:

$$HRh(CO)(XR_3)_2(YR_3)$$

in which R is phenyl; and X and Y are each phosphorus, arsenic or antimony and are different except that both may be antimony, which comprises contacting a complex (a) represented by the general formula,

$$RhZ(CO)(XR_3)_2$$

in which Z is a chloride (except when X and Y are both antimony), bromide or iodide anion with a ligand (b) represented by the general formula,

$$YR_3$$

X, Y and R being as defined above, in a solvent, and adding a reducing agent comprising a hydride of a Group IIIA element at a temperature below room temperature, thereby reacting the mixture to form the desired compound.

Thus, applicants have found that the novel compounds can be obtained in high selectivity and high yield by first contacting a compound of the general formula $RhZ(CO)(XR_3)_2$ in which Z is a halide anion selected from chloride, bromide and iodide, and X and R have the meanings given with a compound of the general formula $YR_3$ in which Y and R have the meanings given in a selected amount depending on the above compound, in a solvent, then adding a reducing agent carefully at a relatively low temperature and thereby reacting them.

The molar ratio of $YR_3$ to $RhZ(CO)(XR_3)_2$ used in the process of the present invention, depends on the properties of the starting materials and the rhodium compound to be formed. In order to obtain the desired compound in a pure form, it is desirable to use $YR_3$ in at least the equimolar amount. When using $YR_3$ in too large in excess, however, the desired compound is obtained in a mixed state with the ligand, as a result of which a number of washings with a solvent will be needed. This is disadvantageous economically and, accordingly, the molar ratio is preferaly 1.0 to 10.

Addition of reducing agents before the contacting of $RhZ(CO)(XR_3)_2$ with $YR_3$ is not desirable because various reactions are thereby caused to take place resulting in deterioration of the reactants. Therefore it is very important to contact the two, suitably by the use of solvents, before adding a reducing agent.

In the process of the invention, any solvents may be used which are capable of dissolving, at least partly, $RhZ(CO)(XR_3)_2$ and/or $YR_3$ but do not react with them. Illustrative of such solvents are aromatic hydrocarbons such as benzene, toluene and lower alcohols such as methanol, ethanol, propanol, isopropanol.

Preferred solvents are lower alcohols, and above all, ethanol may be used in view of advantages in practical operation.

The temperature of contact of $RhZ(CO)(XR_3)_2$ and $YR_3$ can be varied widely within the range in which they are not decomposed, but preferably ranges from room temperature to about 100°C, most preferably from about 40 to about 80°C.

Most useful examples of the reducing agent used in the present invention are alkali metal borohydrides such as sodium borohydride, $NaBH_4$, potassium borohydride, $KBH_4$, and lithium borohydride; boranes such as borane, $BH_3$, diborane, $B_2H_6$, and tetraborane, $B_4H_{10}$; and hydrides of Group IIIA elements such as aluminum, for example, lithium aluminum hydride. Sodium borohydride is preferred because of its cheapness and ease of handling.

When the reducing agent is added at a high temperature after both the reactants are contacted, the heat of reaction sometimes brings about unfavorable side reactions and, therefore, it is desirable to cool to a temperature lower than room temperature, preferably around 0°C, prior to addition of the reducing agent.

The quantity of reducing agent used in the present invention is not particularly limited since no side reaction occurs even if a large quantity of the reducing agent is added, but, in general, a proportion of 5 to 50 mols to 1 mol of $RhZ(CO)(XR_3)_2$ is sufficient. The reducing agent can be added as a solid to the foregoing mixed solution, but is preferably added in the form of a solution in a lower alcohol, etc.

In order to prevent the rapid generation of heat due to the reducing reaction, it is desirable to add the reducing agent gradually while controlling the temperature and stirring the solution adequately. After the reducing agent has been added, cooling may be stopped and the mixture allowed to return to room temperature. The stirring is preferably continued even after the reducing agent is added. This period of time is not particularly limited but usually 1 to 3 hours is sufficient.

When these procedures are carried out, the desired rhodium compound is precipitated from the solution. The product obtained as a precipitate is separated by filtration and washed several times with a solvent to increase its purity.

In the production of $HRh(CO)(SbR_3)_3$ it is desirable to use a starting material in which Z is a bromide or iodide anion. However, even if Z is a chloride anion, Z can readily be converted into a bromide or iodide anion by reaction with potassium bromide or potassium iodide. Thus, the above described seven rhodium compounds can be obtained in high yield.

The process for the production of and the identification of the compounds according to the present invention are illustrated in detail in the following examples.

Example 1

Synthesis of starting materials

(1) Commercially available reagents were used as to rhodium chloride trihydrate $RhCl_3 \cdot 3H_2O$, triphenylphosphine $PPh_3$, triphenylarsine $AsPh_3$, triphenylstibine $SbPh_3$, sodium borohydride $NaBH_4$ and potassium iodide KI.

(2) Di-u-chloro-tetracarbonyl dirhodium $(RhCl(CO)_2)_2$ was prepared from $RhCl_3 \cdot 3H_2O$ and CO by the method disclosed in Inorganic Synthesis, *8*, 211 (1966).

(3) Carbonyl bis(triphenylphosphine) rhodium chloride $RhCl(CO)(PPh_3)_2$ was prepared from $RhCl_3 \cdot 3H_2O$, $PPh_3$ and aqueous solution of formaldehyde by the method disclosed in Inorganic Synthesis, *11*, 99 (1968).

(4) Carbonyl bis(triphenylarsine) rhodium chloride $RHCl(CO)(AsPh_3)_2$ and carbonyl bis(triphenylstibine) rhodium chloride $RhCl(CO)(SbPh_3)_2$ were prepared from $[RhCl(CO)_2]_2$ of (2) and $AsPh_3$ or $SbPh_3$ by the method disclosed in Journal of Chemical Society (A), 2287 (1966).

(5) Carbonyl bis(triphenylstibine) rhodium iodide $RhI(CO)(SbPh_3)_2$: 2.0 g (2.29 mmol) of $RhCl(CO)(SbPh_3)_2$ of (4) and 0.42 g (2.53 mmol) of KI were charged in a 200 ml Erlenmeyer flask, to which 150 ml of acetone as a solvent was then added, and the mixture was reacted with agitation at room temperature for 7 hours. Thereafter, the reaction product was filtered, and the product retained on the filter paper was washed three times with acetone and dried under reduced pressure to obtain 1.7 g of the desired, black-brown compound.

Synthesis of new rhodium compounds

(A) $HRh(CO)(AsPh_3)(PPh_3)_2$

0.5 g (0.724 mmol) of $RhCl(CO)(PPh_3)_2$ of the foregoing (3) and 0.443 g (1.447 mmol) of $AsPh_3$ were charged to a 300 ml three-necked flask, to which 150 ml of ethanol as a solvent was then added, and the mixture was reacted with agitation at a temperature of 65°C in a nitrogen stream for 2 hours. Then the flask was immersed in an ice bath to keep the temperature at 0°C and the mixture was further stirred; to it a solution of 0.5 g (13.21 mmol) of $NaBH_4$ in 50 ml of ethanol was gradually added dropwise over a period of about 30 minutes. Thereafter the flask was withdrawn from the ice bath and the mixture was stirred at room temperature for 2 hours and subjected to filtration in a nitrogen stream. The product retained on the filter paper was washed three times with ethanol and dried under reduced pressure to obtain 0.60 g (0.623 mmol) of a yellow powder (Yield: 86 mol %).

(B) $HRh(CO)(SbPh_3)(PPh_3)_2$

The procedure of the above described (A) was repeated except using 0.511 g (1.447 mmol) of $SbPh_3$ instead of $AsPh_3$ of (A), thus obtaining 0.71 g (0.703 mmol) of a yellow powder (Yield: 97 mol %).

(C) $HRh(CO)(PPh_3)(AsPh_3)_2$

The procedure of the above described (A) was repeated except using 0.5 g (0.642 mmol) of $RhCl(CO)(AsPh_3)_2$ of the foregoing (4) in place of $RhCl(CO)(PPh_3)_2$ of (A) and 0.253 g (0.965 mmol) of $PPh_3$ in place of $AsPh_3$, thus obtaining 0.53 g (0.527 mmol) of a yellow powder (Yield: 82 mol %).

(D) $HRh(CO)(SbPh_3)(AsPh_3)_2$

The procedure of the above described (A) was repeated except using 0.5 g (0.642 mmol) of $RhCl(CO)(AsPh_3)_2$ of the foregoing (4) in place of $RhCl(CO)(PPh_3)_2$ of (A) and 0.34 g (0.963 mmol) of $SbPh_3$ in place of $AsPh_3$, thus obtaining 0.64 g (0.583 mmol) of a yellow-ochre powder (Yield: 91 mol %).

(E) $HRh(CO)(PPh_3)(SbPh_3)_2$

The procedure of the above described (A) was repeated except using 0.5 g (0.573 mmol) of $RhCl(CO)(SbPh_3)_2$ of the foregoing (4) in place of $RhCl(CO)(PPh_3)_2$ of (A) and 0.301 g (1.148 mmol) of $PPh_3$ in place of $AsPh_3$, thus obtaining 0.511 g (0.464 mmol) of a light brown powder (Yield: 81 mol %).

(F) $HRh(CO)(AsPh_3)(SbPh_3)_2$

The procedure of the above described (A) was repeated except using 0.5 g (0.573 mmol) of $RhCl(CO)(SbPh_3)_2$ of the foregoing (4) in place of $RhCl(CO)(PPh_3)_2$ of (A) and 0.35 g (1.143 mmol) of $AsPh_3$, thus obtaining 0.40 g (0.350 mmol) of a brown powder (Yield: 61 mol %).

(G) $HRh(CO)(SbPh_3)_3$

The procedure of the above described (A) was repeated except using 0.5 g (0.519 mmol) of $RhI(CO)(SbPh_3)_2$ of the foregoing (5) in place of $RhCl(CO)(PPh_3)_2$ of (A) and 0.367 g (1.039 mmol) of $SbPh_3$ in place of $AsPh_3$, thus obtaining 0.29 g (0.243 mmol) of a black-brown powder (Yield: 47 mol %).

The decomposition temperatures and IR data (infrared spectra) of the compounds obtained by the above described procedures (A) to (G) are shown in Table 1, the results of elementary analysis are shown in Table 2 and the relationships between the stretching frequencies of Rh-H and C=O of IR ($\upsilon_{Rh-H}$ and $\upsilon_{C=O}$) and the sum of the electronegativities of P, As and Sb in the compounds are shown in Figure 1 and Figure 2. As is apparent from these tables and figures, the compounds of the present invention are all single compounds.

TABLE 1

| Rhodium compound | Color | Decomposition temperature[2] (°C) | IR Data[3,4] ($cm^{-1}$) $\upsilon_{Rh-H}$ | $\upsilon_{C=O}$ |
|---|---|---|---|---|
| $HRh(CO)(AsPh_3)(PPh_3)_2$ | Yellow | 116—118 | 2024(Sh), 2000(S) | 1919(S) |
| $HRh(CO)(SbPh_3)(PPh_3)_2$ | Yellow | 131—133 | 1994(S) | 1913(S) |
| $HRh(CO)(PPh_3)(AsPh_3)_2$ | Yellow | 113—115 | 2010(Sh), 1998(S) | 1916(S) |
| $HRh(CO)(SbPh_3)(AsPh_3)_2$ | Yellow-ochre | 132—134 | 1988(S) | 1897(W) |
| $HRh(CO)(PPh_3)(SbPh_3)_2$ | Light brown | 126—128 | 1994(S) | 1918(M) |
| $HRh(CO)(AsPh_3)(SbPh_3)_2$ | Brown | 143—145 | 1985(S) | 1892(W) |
| $HRh(CO)(SbPh_3)_3$ | Black-brown | 168—171 | 1982(S) | 1884(W) |
| $HRh(CO)(PPh_3)_3$* | Yellow | 137—138 | 2040(S), 2005(S) | 1923(S) |
| $HRh(CO)(AsPh_3)_3$* | Yellow | 130—132 | 1992(S) | 1903(S) |

Remarks: *=Known Compound
2) Decomposition Temperature corresponds to the ordinary melting point since these compounds all melt with decomposition.
3) IR Data were measured in the form of a tablet with KBr and calibrated by CO.
4) Letters in parentheses in IR Data show the absorption intensity of peak:
S=strong; M=medium; W=weak; Sh=shoulder.

TABLE 2

| Rhodium compound | Found (%) | | | | | | Calculated (%) | | | | | |
|---|---|---|---|---|---|---|---|---|---|---|---|---|
| | C | H | P | As | Sb | Rh | C | H | P | As | Sb | Rh |
| $HRh(CO)(AsPh_3)(PPh_3)_2$ | 68.61 | 4.92 | 6.29 | 7.88 | — | 10.84 | 68.62 | 4.82 | 6.43 | 7.78 | — | 10.69 |
| $HRh(CO)(SbPh_3)(PPh_3)_2$ | 65.20 | 4.58 | 6.17 | — | 12.22 | 10.25 | 65.43 | 4.59 | 6.14 | — | 12.06 | 10.19 |
| $HRh(CO)(PPh_3)(AsPh_3)_2$ | 65.73 | 4.76 | 3.31 | 14.72 | — | 10.33 | 65.62 | 4.61 | 3.08 | 14.88 | — | 10.22 |
| $HRh(CO)(SbPh_3)(AsPh_3)_2$ | 59.96 | 4.09 | — | 13.80 | 11.37 | 9.52 | 60.19 | 4.23 | — | 13.65 | 11.09 | 9.30 |
| $HRh(CO)(PPh_3)(SbPh_3)_2$ | 60.07 | 4.25 | 3.00 | — | 21.89 | 9.38 | 60.03 | 4.22 | 2.82 | — | 22.13 | 9.35 |
| $HRh(CO)(AsPh_3)(SbPh_3)_2$ | 57.52 | 3.94 | — | 6.30 | 21.53 | 9.08 | 57.73 | 4.05 | — | 6.55 | 21.28 | 8.99 |
| $HRh(CO)(SbPh_3)_3$ | 55.19 | 3.74 | — | — | 30.57 | 8.88 | 55.46 | 3.89 | — | — | 30.67 | 8.64 |

Comparative Example 1

Using starting materials, solvent and quantity of reducing agent similar to those of Example 1(B), a mixture of $RhCl(CO)(PPh_3)_2$ and $SbPh_3$ in ethanol was held at the boiling temperature, to which an ethanol solution of $NaBH_4$ was added dropwise, and the resulting boiling mixture was further stirred for 3 hours. The starting rhodium compound was decomposed and completely dissolved to give a uniform brown solution only. None of the desired compound was obtained.

Comparative Example 2

When the procedure of Comparative Example 1 was repeated except that starting materials, solvent and quantity of reducing agent were similar to those of Example 1(E), the starting rhodium compound was decomposed and none of the desired compound was obtained.

Comparative Example 3

When the same procedure was repeated except that, in place of $NaBH_4$ in Example 1(E), an aqueous solution of hydrazine in an equimolar quantity thereto was used, only $RhCl(CO)(PPh_3)_2$ was obtained through complete exchange of the ligand.

Comparative Example 4

0.45 g (0.490 mmol) of $HRh(CO)PPh_3)_3$ and 0.173 g (equimolar thereto), 0.346 g (two times mol) or 1.73 g (ten times mol) of $SbPh_3$ were added to 200 ml of ethanol and the resulting mixture was held for 1 hour at the boiling point of ethanol. In each case the starting rhodium compound was only decomposed and none of the compounds aimed at, respectively $HRh(CO)(PPh_3)_2(SbPh_3)$, $HRh(CO)(PPh_3)(SbPh_3)_2$ and $HRh(CO)(SbPh_3)_3$ was obtained.

Comparative Example 5

When the procedure of Comparative Example 4 was repeated except that a temperature of 65°C was employed instead of the boiling temperature of ethanol and stirring continued for 4 hours, only the starting materials with some decomposed product were recovered, which compositions were not clear, and the desired compounds could not be obtained.

Comparative Example 6

When the procedure of Example 1(G) was repeated except that $RhCl(CO)(SbPh_3)_2$ was used instead of $RhI(CO)(SbPh_3)_2$, only the starting materials were recovered.

Comparative Example 7

0.130 g (0.498 mmol) of $RhCl_3 \cdot 3H_2O$, 1.054 g. (corresponding to 6 times mol) of $SbPh_3$ and 3 ml of an aqueous solution of formaldehyde were added to 150 ml of ethanol and the mixture was stirred at the boiling temperature of ethanol in a nitrogen atmosphere, to which mixture a solution of 0.188 g (4.95 mmol) of $NaBH_4$ dissolved in ethanol was added dropwise, followed by refluxing for 3 hours. The starting rhodium compound was decomposed but no $HRh(CO)(SbPh_3)_3$ product was obtained.

It will clearly be seen from a comparison of Example 1 with the Comparative Examples that the preparation process of the present invention is superior and commercially valuable.

The novel rhodium compounds of the present invention are not only effective as a catalyst for the oxo reaction, but are also usable as a catalyst for the hydrogenation of unsaturated organic compounds, the isomerization of double bonds of olefins, the conversion of hydrogen to deuterium, hydrosilylation, and for various other uses.

The advantages of the compounds of the present invention are illustrated by the use as a catalyst for the oxo reaction with propylene as a raw material, the oxo reaction being carried out by the following procedure.

Example 2

20 ml of n-dodecane and 0.109 mmol of a compound of the present invention prepared as described in Example 1 were charged to a 300 ml stainless steel autoclave, which was purged with nitrogen. 5.0 g (119 mmol) of propylene was introduced under pressure into the autoclave which was then heated to 110°C, an $H_2/CO$ gas with a molar ratio of 1/1 being introduced, and the reaction was carried out with the reaction pressure maintained at 20 $Kg/cm^2$. The time when the $H_2/CO$ gas was no longer absorbed was regarded as the final point of the reaction. After the reaction, the reaction product was subjected to analysis by gas chromatography, giving the results shown in Table 3.

It is apparent from the results of Table 3 that the rhodium compound of the present invention is very effective as a catalyst for the oxo reaction.

Certain of the novel compounds according to the present invention have been found to be useful as sources of rhodium in catalyst systems for processes for reacting an olefin with hydrogen and carbon monoxide to produce an aldehyde having one more carbon atom than the starting olefin. The catalyst systems comprise a rhodium source and free mixed ligands comprising a tertiary organo phosphorus and tertiary organo arsenic represented by the general formula $X'R''_3$ (where X' represents phosphorus or

arsenic and R'' represents an organic group, which may be the same or different) in excess of the quantity required for coordination to the rhodium atom. Such processes form the subject of our copending European patent application 79302093.4 (publication No. 0014 796), out of which this present application is divided.

TABLE 3

| Rhodium compound | Reaction rate $-d(H_2+CO)/dt$ (ml/sec) | Reaction time (min) | Conversion of propylene (mol %) | Yield of butylaldehyde (mol %) | n/i Ratio of butylaldehyde | Yield of propane (mol %) |
|---|---|---|---|---|---|---|
| $HRh(CO)(AsPh_3)(PPh_3)_2$ | 13.5 | 13 | 90.8 | 89.8 | 2.0 | 0.7 |
| $HRh(CO)(SbPh_3)(PPh_3)_2$ | 1.5 | 100 | 43.0 | 42.1 | 1.9 | 0.4 |
| $HRh(CO)(PPh_3)(AsPh_3)_2$ | 10.2 | 30 | 89.5 | 88.6 | 2.1 | 0.6 |
| $HRh(CO)(SbPh_3)(AsPh_3)_2$ | 0.5 | 300 | 19.7 | 18.3 | 2.1 | 0.2 |
| $HRh(CO)(PPh_3)(SbPh_3)_2$ | 1.2 | 275 | 50.4 | 49.8 | 2.0 | 0.4 |
| $HRh(CO)(AsPh_3)(SbPh_3)_2$ | 0.4 | 300 | 17.7 | 16.8 | 2.2 | 0.3 |
| $HRh(CO)(SbPh_3)_3$ | 0.2 | 300 | 7.5 | 7.0 | 2.3 | 0.3 |

## Claims

1. Rhodium compounds having the general formula,

$$HRh(CO)(XR_3)_2(YR_3)$$

in which X and Y are different and are each phosphorus, arsenic, or antimony; and R is phenyl.

2. The compound according to claim 1 having the formula:

$$HRh(CO)(PPh_3)_2(AsPh_3)$$

3. The compound according to claim 1 having the formula:

$$HRh(CO)(PPh_3)(AsPh_3)_2$$

4. The compound according to claim 1 having the formula:

$$HRh(CO)(PPh_3)_2(SbPh_3)$$

5. The compound according to claim 1 having the formula:

$$HRh(CO)(PPh_3)(SbPh_3)_2$$

6. The compound according to claim 1 having the formula:

$$HRh(CO)(AsPh_3)_2(SbPh_3)$$

7. The compound according to claim 1 having the formula:

$$HRh(CO)(AsPh_3)(SbPh_3)_2$$

8. A process for producing a rhodium compound having the general formula:

$$HRh(CO)(XR_3)_2(YR_3)$$

in which R is phenyl; and X and Y are each phosphorus, arsenic or antimony and are different except that both may be antimony, which comprises contacting a complex (a) represented by the general formula,

$$RhZ(CO)(XR_3)_2$$

in which Z is chloride (except when X and Y are both antimony), bromide or iodide anion with a ligand (b) represented by the general formula,

$$YR_3$$

X, Y and R being as defined above, in a solvent, and adding a reducing agent comprising a hydride of a Group IIIA element at a temperature below room temperature, thereby reacting the mixture to form the desired compound.

9. A process according to claim 8 wherein (b) and (a) are contacted in a molar ratio of 1.0:1 to 10:1.

10. A process according to claim 8 wherein the reducing agent is an alkali metal borohydride.

11. The use of a rhodium compound according to any one of claims 1 to 7, or produced by the process according to claim 8, 9 or 10, as an oxo-reaction catalyst.

## Patentansprüche

1. Rhodiumverbindungen der allgemeinen Formel

$$HRh(CO)(XR_3)_2(YR_3),$$

in welcher X und Y verschieden sind und jeweils Phosphor, Arsen oder Antimon bedeuten und R ein Phenylrest ist.

2. Die Verbindung gemäß Anspruch 1 mit der Formel

$$HRh(CO)(PPh_3)_2(AsPh_3)$$

3. Die Verbindung gemäß Anspruch 1 mit der Formel

$$HRh(CO)(PPh_3)(AsPh_3)_2$$

4. Die Verbindung gemäß Anspruch 1 mit der Formel

$$HRh(CO)(PPh_3)_2(SbPh_3)$$

5. Die Verbindung gemäß Anspruch 1 mit der Formel

$$HRh(CO)(PPh_3)(SbPh_3)_2$$

6. Die Verbindung gemäß Anspruch 1 mit der Formel

$$HRh(CO)(AsPh_3)_2(SbPh_3)$$

7. Die Verbindung gemäß Anspruch 1 mit der Formel

$$HRh(CO)(AsPh_3)(SbPh_3)_2$$

8. Verfahren zur Herstellung einer Rhodiumverbindung der allgemeinen Formel

$$HRh(CO)(XR_3)_2(YR_3),$$

in der R ein Phenylrest ist und X und Y jeweils Phosphor, Arsen oder Antimon bedeuten und verschieden sind mit der Ausnahme, daß beide Antimon sein können, wobei das Verfahren darin besteht, daß man eine Komplexverbindung

(a) entsprechend der allgemeinen Formel

$$RhZ(CO)(XR_3)_2,$$

in der Z ein Chloridanion (ausgenommen wenn X und Y beide Antimon sind), Bromid- oder Jodidanion ist, in einem Lösungsmittel mit einem Liganden (b) entsprechend der allgemeinen Formel $YR_3$ in Kontakt bringt, wobei X, Y und R die oben angegebene Bedeutung haben; und bei einer Temperatur unterhalb Zimmertemperatur ein Reduktionsmittel zusetzt, bei dem as sich um ein Hydrid eines Elementes der Gruppe IIIA handelt, wodurch die Mischung unter Bildung der gewünschten Verbindung reagiert.

9. Verfahren gemäß Anspruch 8, bei welchem (b) und (a) in einem Molverhältnis von 1,0:1 bis 10:1 miteinander in Kontakt gebracht werden.

10. Verfahren gemäß Anspruch 8, bei welchem das Reduktionsmittel ein Alkaliborhydrid ist.

11. Verwendung einer Rhodiumverbindung gemäß einem der Ansprüche 1 bis 7 oder einer nach dem Verfahren gemäß den Ansprüchen 8, 9 oder 10 hergestellten Rhodiumverbindung als Katalysator für die Oxosynthese.

**Revendications**

1. Composés de rhodium répondant à la formule générale

$$HRh(CO)(XR_3)_2(YR_3)$$

dans laquelle X et Y sont différents et représentent chacun le phosphore, l'arsenic ou l'antimoine; et R est le groupe phényle.

2. Composé suivant la revendication 1, répondant à la formule:

$$HRh(CO)(PPh_3)_2(AsPh_3).$$

3. Composé suivant la revendication 1, répondant à la formule:

$$HRh(CO)(PPh_3)(AsPh_3)_2.$$

4. Composé suivant la revendication 1, répondant à la formule:

$$HRh(CO)(PPh_3)_2(SbPh_3).$$

5. Composé suivant la revendication 1, répondant à la formule:

$$HRh(CO)(PPh_3)(SbPh_3)_2.$$

6. Composé suivant la revendication 1, répondant à la formule:

$$HRh(CO)(AsPh_3)_2(SbPh_3).$$

7. Composé suivant la revendication 1, répondant à la formule:

$$HRh(CO)(AsPh_3)(SbPh_3)_2.$$

8. Procédé de production d'un composé de rhodium répondant à la formule générale:

$$HRh(CO)(XR_3)_2(YR_3)$$

dans laquelle R est un groupe phényle; et X et Y représentent chacun le phosphore, l'arsenic ou l'antimoine et sont différents, excepté que tous deux peuvent représenter de l'antimoine, qui consiste à faire entrer un complexe (a) représenté par la formule générale

$$RhZ(CO)(XR_3)_2$$

dans laquelle Z est un anion chlorure (excepté lorsque X et Y sont tous deux l'antimoine), bromure ou iodure, en contact avec un ligand (b) représenté par la formule générale

$$YR_3$$

X, Y et R ayant la définition donnée ci-dessus, dans un solvant, et à ajouter un agent réducteur comprenant un hydrure d'un élément du Groupe IIIA à une température inférieure à la température ambiante, en faisant ainsi réagir le mélange pour former le composé désiré.

9. Procédé suivant la revendication 8, dans lequel les composés (b) et (a) sont mis en contact dans un rapport molaire de 1,0:1 à 10:1.

10. Procédé suivant la revendication 8, dans lequel l'agent réducteur est un borohydrure de métal alcalin.

11. Utilisation d'un composé de rhodium suivant l'une quelconque des revendications 1 à 7, ou produit par le procédé suivant la revendication 8, 9 ou 10, comme catalyseur d'une réaction oxo.

FIG. 1

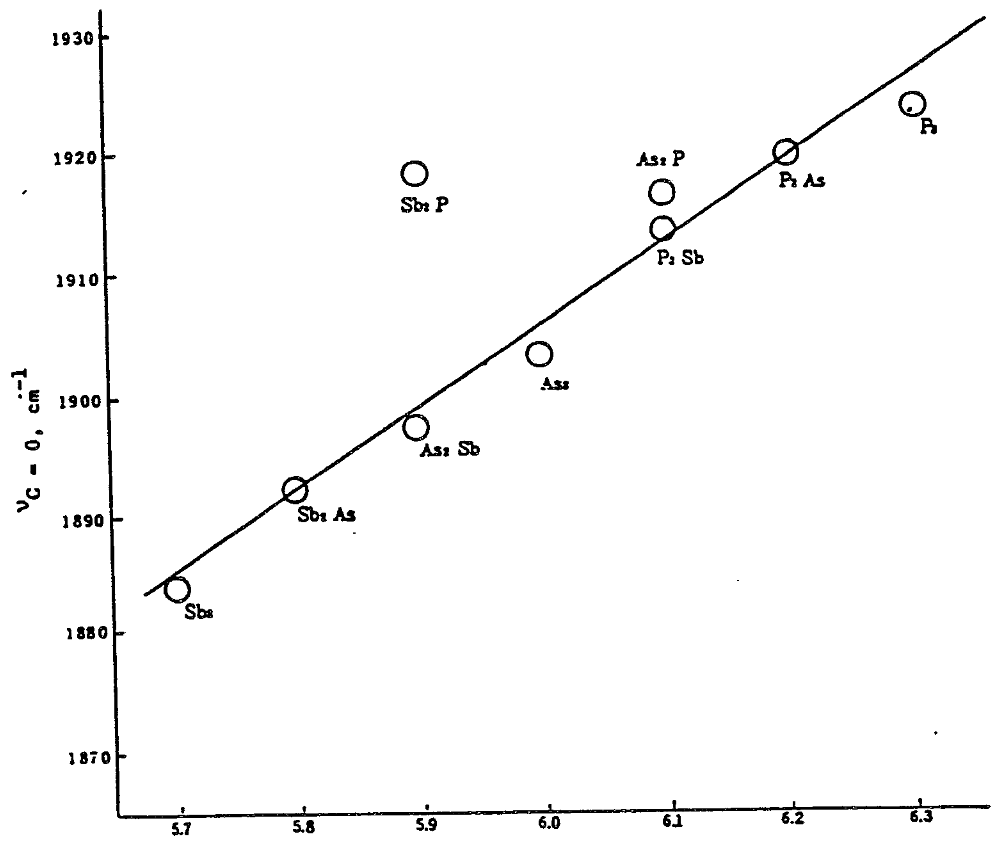


Sum of the Pauling electro-negativities (Xp)

FIG. 2

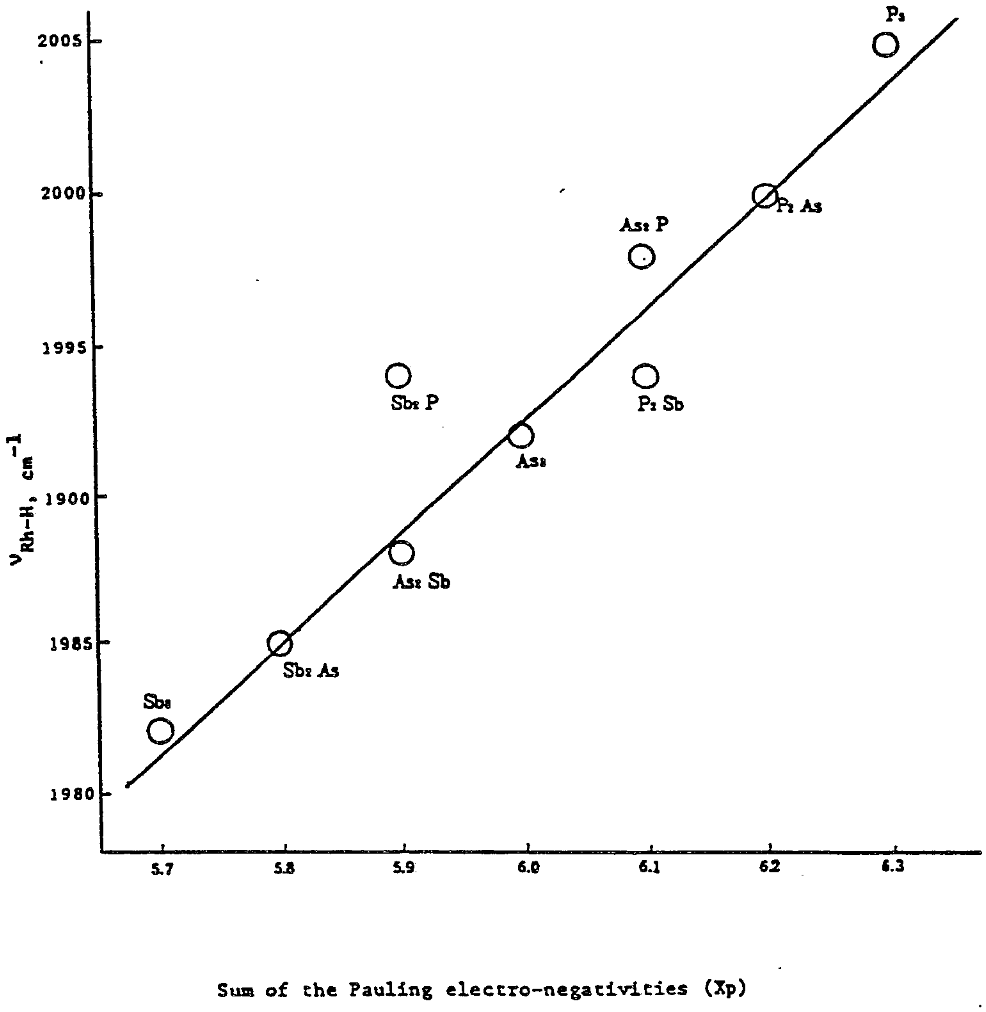
P3
2005
2000
P2 As
As2 P
1995
Sb2 P
P2 Sb
As3
1900
$\nu_{Rh-H}$, $cm^{-1}$
As2 Sb
1985
Sb2 As
Sb3
1980
5.7
5.8
5.9
6.0
6.1
6.2
6.3
Sum of the Pauling electro-negativities (Xp)